(19) Europäisches Patentamt
European Patent Office
Office européen des brevets

(11) Numéro de publication: **0 069 674**
**B1**

# (12) FASCICULE DE BREVET EUROPEEN

(45) Date de publication du fascicule du brevet: **17.04.85**

(51) Int. Cl.⁴: **C 07 C 143/155,** A 61 K 31/195

(21) Numéro de dépôt: **82401280.1**

(22) Date de dépôt: **07.07.82**

## (54) Médicaments contenant une N-acyl-L-aspartyl-taurine.

(30) Priorité: **09.07.81 CH 4514/81**

(43) Date de publication de la demande: **12.01.83 Bulletin 83/2**

(45) Mention de la délivrance du brevet: **17.04.85 Bulletin 85/16**

(84) Etats contractants désignés: **AT BE CH DE FR GB IT LI LU NL SE**

(56) Documents cités:
**FR - A - 2 257 270**
**FR - A - 2 279 388**

(73) Titulaire: **Société à Responsabilité Limitée dite : B F B Etudes et Recherches Expérimentales, 9, rue de Nemours, F-75011 Paris (FR)**

(72) Inventeur: **Flork, Michel, 63 route d'Aubusson, F-63380 Pontaumur (FR)**
Inventeur: **Bigou, Alphonse, 60 avenue Paul Doumer, F-75016 Paris (FR)**

(74) Mandataire: **Combe, André et al, CABINET BEAU DE LOMENIE 55 rue d'Amsterdam, F-75008 Paris (FR)**

**EP 0 069 674 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne les N-acyl-L-aspartyl-taurine et les médicaments contenant lesdits produits.

La taurine (acide amino-2-éthane sulfonique) est un acide aminé qui semble jouer un rôle important dans les mécanismes biologiques. De ce fait, on a mentionné récemment, que la taurine pourrait avoir des propriétés pharmacologiques intéressantes, notamment des effets neurotransmetteurs (elle agirait comme modulateur de la neurotransmission), des effets régulateurs des transmissions d'ions à travers les membranes cellulaires, des effets trophiques, de sorte que l'on a envisagé l'emploi de taurine comme agent anti-épileptique, comme agent anti-arythmique et pour le traitement de l'hypertension. Cependant il est actuellement très difficile d'utiliser effectivement la taurine et ses dérivés connus comme médicaments, car on n'est pas encore parvenu à trouver un moyen permettant d'atteindre une concentration locale, en taurine, suffisante pour que cet aminoacide puisse avoir une action décisive. C'est là le but de la présente invention.

D'autre part, il est connu du brevet francais 2 257 270 que le N-acétyl-L-aspartyl-glutamate agit sur le système Merveux central et possède des propriétes cardiovasculaires.

Il est également connu du brevet francais 2 279 388 que l'aspartyl-taurine et la N-acétyl-glutamyl-taurine sont utilisées en thérapeutique.

Maintenant il a été trouvé qu'en liant la taurine (ou un de ses sels) au reste N-acyl-L-aspartyl, ce reste jouait le rôle d'un vecteur capable de véhiculer la taurine jusqu'à l'organe où son mode d'action est souhaité (ledit organe étant très vraisemblablement le système nerveux central) permettant ainsi, au niveau de cet organe, de bénéficier des effets d'une concentration suffisante et se maintenant suffisamment longtemps.

Comme vecteur, on utilise selon la présente invention une N-acyl-L-aspartyl et plus précisément la N-acétyl-L-aspartyl.

La préparation d'ure N-acyl-L-aspartyl taurine et plus particulièrement du N-acétyl-L-aspartyl taurine peut s'effectuer en faisant réagir vers 0° C, des quantités sensiblement équimoléculaires de taurine (en solution aqueuse basique) et d'anhydride de l'acide N-acétyl-L-aspartique.

Le produit obtenu possède des propriétés pharmacologiques remarquables qui en font un principe actif utilisable en cardiologie (antiarythmique et antiangineux), en neurologie (anticomitial et anxiolytique), en ophtalmologie (action positive sur certains récepteurs de la rétine), en nutrition (facteur de croissance tissulaire et alimentation entérale) et surtout comme antihypertenseur.

La mise en évidence de l'effet antihypertenseur du produit selon l'invention, a été effectuée sur le rat spontanément hypertendu; le produit a été administré par voie orale, avec la nourriture, à raison de 1% en poids pendant trois semaines chez dix animaux et les résultats obtenus ont été comparés avec un lot d'animaux témoins.

Il apparait dès le cinquième jour du traitement, que les rats ayant reçu le principe actif, présentent une tension artérielle significativement plus faible que la tension des rats témoins. Dans les jours qui suivent, on constate, après une baisse très accentuée de la tension, une remontée régulière de cette tension qui reste toujours cependant plus basse — et de façon nette — que la tension des rats témoins. On en conclut que le produit actif a d'une part un effet antihypertenseur très net et d'autre part un effet retardateur du développement de l'hypertension.

Le produit selon l'invention est utilisable par voie orale à raison de 0,5 à 1,5 g par jour réparti en une ou plusieurs prises.

Bien évidemment, le produit selon l'invention ne présente comme prévu, aucune toxicité.

L'exemple non limitatif ci-après illustre le procédé de préparation du N-acétyl-aspartyl-taurine.

Exemple

On réalise l'acylation de la taurine par l'anhydride N-acétyl-L-aspartique selon le schéma et la technique ci-après:

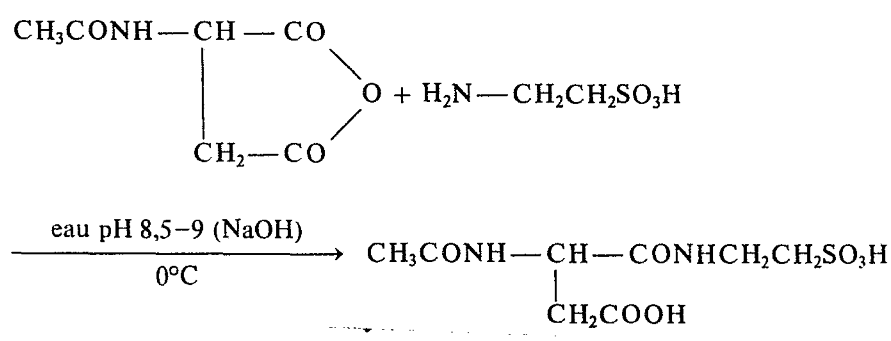


Dissoudre 1,05 mole de taurine dans 500 ml d'eau en ajustant le pH à 9,0 par addition de lessive de

soude 10 N. Refroidir cette solution à 0° puis ajouter sous agitation vigoureuse, 1 mole d'anhydride N-acétyl-L-aspartique, en une heure, par petites portions (Conserver cet anhydride à l'abri de l'humidité avant l'addition). Agiter encore 10 mn après la fin de l'addition. Le pH est mesuré en permanence, et maintenu entre 8,5 et 9 par addition d'une solution de soude 4 N.

La solution obtenue est ensuite percolée sur résine cationique sulfonique (type C-20 marque »Duolite«). Le percolat est concentré sous vide jusqu'à un volume de 400 ml. Le sirop obtenu cristallise. La suspension est filtrée après 24 h de cristallisation, lavée par l'acétone, et séchée. On obtient 0,65 mole d'acide N-acétyl-L-aspartyl-taurine.

**Revendications pour les Etats contractants: BE, CH, DE, FR, GB, IT, LI, LU, NL, SE**

1. Médicaments présentant notamment l'activité d'un antihypertenseur, caractérisé en ce qu'il contient, comme principe actif, une N-acyl-L-aspartyl taurine.
2. Médicament selon la revendication 1, caractérisé en ce que le principe actif est la N-acétyl-L-aspartyl taurine.
3. Compositions pharmaceutiques renfermant à titre de principe actif une N-acyl-L-aspartyl taurine en association ou en mélange avec un excipient ou un véhicule inerte non toxique, pharmaceutiquement acceptable.

**Revendications pour l'Etat contractant: AT**

1. Procédé de préparation d'une N-acyl-L-aspartyl taurine utilisable notamment dans les médicaments, caractérisé en ce que l'on fait réagir, aux environs de 0° C, des quantités sensiblement équimoléculaire de taurine, en solution aqueuse basique, et d'anhydride d'un acide N-acyl-L-aspartique.
2. Procédé selon la revendication 1, caractérisé en ce que l'anhydride de l'acide N-acyl-L-aspartique est l'anhydride de l'acide N-acétyl-L-aspartique.

**Patentansprüche für die Vertragsstaaten: BE, CH, DE, FR, GB, IT, LI, LU, NL, SE**

1. Medikamente, die insbesondere blutdrucksenkende Aktivität aufweisen, dadurch gekennzeichnet, daß sie als Wirkstoff ein N-Acyl-L-asparaginyl-taurin enthalten.
2. Medikament nach Anspruch 1, dadurch gekennzeichnet, daß der Wirkstoff N-Acetyl-L-asparaginyl-taurin ist.
3. Pharmazeutische Zusammensetzungen, die als Wirkstoff N-Acyl-L-Asparaginyl-taurin in Verbindung oder in Mischung mit einem inerten, nicht toxischen, pharmazeutisch akzeptablen Exzipienten oder Vehikel enthalten.

**Patentansprüche für den Vertragsstaat: AT**

1. Verfahren zur Herstellung eines N-Acyl-L-asparaginyl-taurins, das insbesondere für Medikamente geeignet ist, dadurch gekennzeichnet, daß man bei etwa 0° C im wesentlichen äquimolare Mengen von Taurin in wässeriger basischer Lösung mit einem N-Acyl-L-asparaginsäureanhydrid zur Umsetzung bringt.
2. Verfahren nach Anspruch 1, dadurch gekennzeichnet, daß das N-Acyl-L-asparaginsäureanhydrid N-Acetyl-L-asparaginsäureanhydrid ist.

**Claims for the Contracting States: BE, CH, DE, FR, GB, IT, LI, LU, NL, SE**

1. Drugs having, in particular, an antihypertensive activity, characterized in that it contains, as active ingredient an N-acyl-L-aspartyl taurine.
2. Drug according to claim 1, characterized in that the active ingredient is N-acetyl-L-aspartyl taurine.
3. Pharmaceutical compositions containing as active ingredient an N-acyl-L-aspartyl taurine associated to or mixed with a pharmaceutically acceptable non-toxic inert vehicle or excipient.

**Claims for the Contracting State: AT**

1. Process for the preparation of an N-acyl-L-aspartyl usable particularly in drugs, characterized in that it is prepared by reacting, towards 0° C, substantially equimolecular quantities of taurine, in basic

aqueous solution, and of anhydride of N-acetyl-L-aspartic acid.

2. Process according to claim 1, characterized in that the anhydride of N-acyl-L-aspartic acid is the anhydride of N-acetyl-L-aspartic acid.